# EUROPÄISCHE PATENTSCHRIFT

(11) **EP 1 664 789 B1**
(45) Veröffentlichungstag und Bekanntmachung des Hinweises auf die Patenterteilung: **23.02.2011**
(21) Anmeldenummer: 04764908.2
(22) Anmeldetag: 07.09.2004
(51) Int. Cl.: G01N 33/574

(54) **VERFAHREN ZUR DIAGNOSE VON ERKRANKUNGEN UNTER BESTIMMUNG VON APOLIPOPROTEIN C-I**
METHOD FOR THE DIAGNOSIS OF DISEASES BY DETERMINING APOLIPOPROTEIN C-I
PROCEDE POUR DIAGNOSTIQUER DES MALADIES EN IDENTIFIANT UNE APOLIPOPROTEINE C-I

(30) Priorität: 22.09.2003 DE 10343815
(43) Veröffentlichungstag der Anmeldung: 07.06.2006
(73) Patentinhaber: B.R.A.H.M.S GmbH, 16761 Hennigsdorf (DE)
(72) Erfinder: BERGMANN, Andreas, 12351 Berlin (DE)
(74) Vertreter: Andrae, Steffen
(86) Internationale Anmeldenummer: PCT/EP2004/009965
(87) Internationale Veröffentlichungsnummer: WO 2005/038461

(56) Entgegenhaltungen:
- WO-A-03/076594
- US-A1- 2001 005 714
- BARLAGE S ET AL: "ApoE-containing high density lipoproteins and phospholipid transfer protein activity increase in patients with a systemic inflammatory response." JOURNAL OF LIPID RESEARCH. FEB 2001, vol. 42, no. 2, February 2001 (2001-02), pages 281-290, XP002309046 ISSN: 0022-2275

## Beschreibung

Die vorliegende Erfindung betrifft Verfahren zur Diagnose, Früherkennung, Risikoabschätzung sowie zur Verlaufskontrolle von Erkrankungen, bei denen in Serum- oder Plasmaproben menschlicher Patienten als Biomarker das Protein Apolipoprotein C-I bestimmt wird.

Es ist dabei vorausschickend anzumerken, dass, wenn in der nachfolgenden Beschreibung allgemein von "diagnostischen Verfahren" gesprochen wird, dieser Begriff in der Regel vereinfachend für Verfahren zur Diagnose, Früherkennung, Risikoabschätzung sowie zur Verlaufskontrolle, einschließlich einer therapiebegleitenden Verlaufkontrolle, von Erkrankungen stehen soll, es sei denn, es ergibt sich aus dem konkreten Zusammenhang, dass an der jeweiligen Stelle nur Messungen mit speziellen eingeschränkten Zielsetzungen gemeint sind.

Ferner ist anzumerken, dass im Kontext der vorliegenden Anmeldung der Begriff "Erkrankungen" für in der Regel schwere chronische und insbesondere akute Erkrankungen verwendet wird und sich nicht auf Störungen des Fettstoffwechsels, wie sie als Risikofaktoren für die Entwickung einer Atherosklerose angesehen werden, beziehen soll. Als "Erkrankungen" sind Krebserkrankungen hervorzuheben.

Als "Apolipoproteine" werden Proteine bezeichnet, die gemeinsam mit Lipiden (Triglyceriden, Cholesterin, Phospholipiden) die Lipoproteine bilden. Eine wichtige Funktion der Apolipoproteine ist es, den Transport der wasserunlöslichen Lipide in Serum und Plasma zu ermöglichen. Lipoproteine werden aufgrund ihres Wanderungsverhaltens im Dichtegradienten einer Ultrazentrifuge vier verschiedenen Dichteklassen zugeordnet: Chylomikronen, VLDL (von: very low-density lipoproteins), LDL (von: low-density lipoproteins) und HDL (von: high-density lipoproteins). Zusätzlich unterscheidet man eine als Lipoprotein(a) bezeichnete Gruppe. Der Lipidanteil der Lipoproteine ist um so geringer, je höher deren Dichte ist. Lipoproteine unterschiedlicher Dichteklassen unterscheiden sich nicht nur aufgrund der Menge des gesamten vorhandenen Lipidanteils, sondern auch im Hinblick auf dessen Zusammensetzung. Den jeweiligen Dichteklasse lassen sich ferner auch typische Apolipoproteinprofile zuordnen.

Apolipoproteine sind Proteine unterschiedlicher Länge und Aminosäurezusammensetzung, die mit Großbuchstaben A-H bezeichneten Gruppen zugeordnet werden, wobei innerhalb der einzelnen Gruppen nochmals zwischen unterschiedlichen Proteinen unterschieden wird, die durch eine angefügte Zahl charakterisiert werden. An Stelle der vollständigen Bezeichnung von z.B. Apolipoprotein A-I hat es sich eingebürgert, auch einfach nur von Apo A-I zu sprechen. Die Primärstrukturen (Aminosäuresequenzen) der verschiedenen Apolipoproteine sind bekannt, und für eine Reihe der verschiedenen Apolipoproteine existieren auch Daten oder konkrete Vorstellungen zu ihrer räumlichen Struktur, insbesondere in Assoziation mit Lipiden. Genauere Angaben sind der einschlägigen wissenschaftlichen Literatur entnehmbar.

Es ist bekannt, im Zusammenhang mit der Gewinnung von Meßdaten zum Fettstoffwechsel eines Patienten, insbesondere zur Früherkennung eines Atheroskleroserisikos oder zur Therapie-Kontrolle bei der Behandlung eines Patienten mit lipidsenkenden Medikamenten, auch gezielt Apolipoproteine zu bestimmen. Dabei macht man sich den Umstand zunutze, dass bestimmte Apolipoproteine ausschließlich oder wenigstens überwiegend in Lipoproteinen der einzelnen der oben genannten Lipoprotein-Dichteklassen auftreten und deren Spezifität bestimmen, so dass man durch eine Bestimmung des Apolipoproteinteils von Lipoproteinen auch den Anteil der letzteren in einem Serum oder Plasma eines Patienten ermitteln kann.

Es ist auch bekannt, dass bei einzelnen Patienten aufgrund erblicher Einflüsse, die die Bildung der Apolipoproteine oder der diese spaltenden Enzyme beeinflussen oder sich als Rezeptordefekte äußern, vom Normalfall abweichende Lipoprotein- und auch Apolipoproteinprofile gefunden werden. Es ist ferner bekannt, dass es auch im Gefolge anderer Erkrankungen zu Fettstoffwechselstörungen (sekundären Dyslipoproteinämien) kommen kann, die temporärer Natur sind und nach Heilung der Erkrankung wieder verschwinden (vgl. (20), Seiten 186-189; Literaturverweise in Form von Zahlen in Klammern beziehen sich auf das beigefügte Literaturverzeichnis). Soweit die Veränderungen des Fettstoffwechsels sich auch als Veränderungen der Apolipoprotein-Zusammensetzungen äußern, sind in der wissenschaftlichen Literatur in erster Linie solche Veränderungen dokumentiert, die die Apolipoproteine A-I und A-II betreffen und in der Regel abnorme Formen und Konzentrationen der HDL widerspiegeln. Als Biomarker für die Diagnose von Erkrankungen, für die bekannt ist, dass sie auch von Fettstoffwechselstörungen begleitet sind, spielen Apolipoproteine jedoch keine nennenswerte praktische Rolle.

Im Rahmen der vorliegenden Anmeldung geht es um die Bestimmung von Apolipoprotein C-I zu diagnostischen Zwecken. Das Apolipoprotein C-I gehört zu einer Gruppe von Apolipoproteinen mit vergleichweise niedrigem Molekulargewicht, die als die Apolipoproteine Apo C-I, Apo C-II und Apo C-III bezeichnet werden. Die Primärstrukturen dieser drei Proteine sind bekannt (vgl. (1), (2) und, im Hinblick auf Apolipoprotein C-I, insbesondere (3)). Über die Funktion der Apolipoproteine C-I und C-III ist relativ wenig bekannt, und über Veränderungen der Mengen bzw. Formen und Assoziationsformen von Apolipoprotein C-I in humanem Serum oder Plasma in Abhängigkeit von bestimmten Krankheitszuständen liegen keine dokumentierten Erkenntnisse vor. Repräsentativ für Publikationen der wissenschaftlichen Literatur, die Apolipoproteine der Gruppe C bzw. Apolipoprotein C-I betreffen, sind Veröffentlichungen gemäß (1) bis (18), wobei insbesondere die Arbeit gemäß (11) eine guten Überblick darüber liefert, was über die Rolle der Apolipoproteine C im Lipoprotein-Stoffwechsel bekannt ist. Zu den für Apolipoprotein C-I beschriebenen biologischen Effekten gehören in erster Linie die Hemmung des Cholesterinester-Transferproteins (CETP), die Hemmung des dem LDL-Rezeptor (LDLR) zugeordneten Proteins (LRP) und des LDLR selbst und des VLDL-Rezeptors (VLDLR) sowie die Aktivierung der Lecithin-Cholesterin-Acyltransferase (LCAT).

Aus dem Inhalt der genannten Veröffentlichungen sind keine Erkenntnisse ableitbar, die eine Bestimmung von Apolipoprotein C-I als Biomarker zur Diagnose von Erkrankungen, wie z.B. Sepsis oder Krebs, ermöglichen oder eine Bestimmung von Apolipoprotein C-I in den genannten diagnostischen Zusammenhängen nahelegen würden.

Die vorliegende Erfindung ist das Ergebnis von systematischen Forschungsarbeiten der Anmelderin, die zum Ziel haben, Veränderungen der Proteinzusammensetzung von Patienten mit Sepsis zu erforschen und die gewonnenen Forschungsergebnisse gezielt für die Sepsis- und Entzündungsdiagnostik nutzbar zu machen.

Ausgangspunkt dieser Forschungen war die Feststellung, dass bei Sepsis das Prohormon Procalcitonin drastisch erhöht ist und dass die Bestimmung von Procalcitonin als Biomarker für Sepsis zur Diagnose und zur therapiebegleitenden Verlaufskontrolle von hohem praktischen Wert ist (vgl. z.B. EP 656 121 B1). Weiterführende Erkenntnisse zur Veränderung der Proteinzusammensetzung im Serum oder Plasma von Sepsispatienten, die bei der Anmelderin ausgehend von unterschiedlichen Forschungsansätzen gewonnen wurden, finden sich beispielsweise in der veröffentlichten Patentanmeldung EP 1 121 600 ,A2 sowie weiteren veröffentlichten Patentanmeldungen jüngeren Datums wie WO02/085937, WO03/005035, WO03/002600, EP 1 355 158 A1, EP 1 318 406 A1, EP 1 318 407 A1 und EP 1 355 159 A1 und zusätzlichen einschlägigen, noch unveröffentlichten Patentanmeldungen der Anmelderin. Auf den Inhalt der genannten Patentanmeldungen wird insbesondere im Hinblick auf die Erläuterungen zum modernen Verständnis des Begriffs Sepsis und die Bedeutung der Bestimmung von Biomarkern bei Sepsis verwiesen.

Da einige der Untersuchungen gezeigt hatten, dass bei Sepsis auch eine Reihe von Biomarkern signifikant erhöht sind, die traditionell nur als typische Krebsmarker angesehen wurden (vgl. EP 1 318 402 A1, EP 1 318 403 A1, EP 1 318 404 A1 und EP 1 318 405 A1) wurden zunehmend parallel zur Untersuchung von Proben von Sepsispatienten auch Plasma- und Serumproben von Patienten berücksichtigt, die an verschiedenen malignen Turmorerkrankungen litten. In vielen der untersuchten Fälle zeigte es sich, dass einzelne Biomarker, die bei Sepsis erhöht gefunden werden, auch bei Krebspatienten signifikant erhöht sind. In zahlreichen Fällen zeigten sich jedoch auch deutliche Unterschiede, die es verbieten, Sepsiserkrankungen und maligne Tumorerkrankungen in einer simplifizierenden Betrachtungsweise generell als grundsätzlich ähnlich zu behandeln.

Die in der vorliegenden Anmeldung beschriebene Erfindung ist Resultat eines weiteren Forschungsansatzes zur Ermittlung von Proteinen, die sich potentiell als Biomarker für diagnostische Zwecke eigenen, weil ihre messbaren Mengen bei Sepsispatienten und ggf. Patienten, die an anderen schweren Erkrankungen, insbesondere entzündlichen Erkrankungen oder Krebserkrankungen, leiden, gegenüber Gesunden signifikant verändert sind. Es wurden dazu HPLC-Profile von Serum- bzw. Plasmafraktionen von Sepsispatienten mit denen von augenscheinlich gesunden Normalpersonen verglichen und signifikante Veränderungen der HPLC-Chromatographie-Profile einer näheren Prüfung unterzogen. Diese Untersuchungen lieferten die Ergebnisse, die zur Grundlage der vorliegenden Erfindung wurden, nämlich dass signifikante Veränderungen in den HPLC-Elutionsprofilen von Serum- oder Plasma-Proben von Patienten erkennbar werden, wenn diese Proben vorher über eine Säule mit einem Chromatographiematerial aufgetrennt wurden, das in der Lage ist, mit hydrophoben bzw. lipophilen Bestandteilen der Probe in Wechselwirkung zu, treten und derartige Bestandteile selektiv zu binden ("hydrophobe Interaktionschromatographie"). Werden die an das Chromatographiematerial gebundenden Bestandteile der aufgetrennten Probe anschließend auf geeignete Weise eluiert, wird eine Subfraktion der ursprünglichen Serum- oder Plasmaprobe erhalten, in der Bestandteile, insbesondere auch solche proteinischer Natur, die in der Lage sind, Wechselwirkungen mit hydrophoben Oberflächen einzugehen, selektiv angereichert sind. Eine Variante eines solchen Verfahrens in Anwendung auf Lipoproteine ist in (19) beschrieben.

In der US 2001/0005714 A1 wird vorgeschlagen, die Tatsache, dass Apolipoproteine in ihrer Struktur amphipathische Abschnitte aufweisen, dazu zu nutzen, die Aufnahme von Cholesterin und anderen Lipiden aus dem Darm zu inhibieren, indem man Apolipoproteine oder andere Moleküle mit amphipathischen Strukturen, die den Apolipoproteinen ähneln, als Medikamente einsetzt. Zur Isolierung von Apolipoproteinen aus natürlichen Quellen wird die hydrophobe Interaktions-Chromatographie eingesetzt.

Die Anmeldung WO 03/076594 offenbart Methoden, um Krebserkrankungen zu diagnostizieren und zu behandeln, indem epigenetisch stillgelegte (Tumorsuppressor)gene detektiert und eventuell reaktiviert werden. Eines der 58 identifizierten, epigenetisch stillgelegten (Tumorsuppressor)gene ist Apo C-I. WO 03/076594 (Par. 55-57) korreliert eine Reihe verschiedener Krebserkrankungen mit solchen epigenetisch stillgelegten (Tumorsuppressor)genen, darunter auch dem Apo C-I Gen.

Die Auftrennung derartiger Fraktionen von Plasmen bzw. Seren von Patienten mit Sepsis und anderen Erkrankungen mittels Hochleistungs-Flüssigkeitschromatographie (HPLC) lieferte die der vorliegenden Erfindung zugrundeliegende überraschende Erkenntnis, dass in den Seren von Sepsis-Patienten und bestimmten anderen Patienten ein Bestandteil, der in Seren und Plasmen gesunder Normalpersonen in deutlicher Ausprägung zu finden ist, stark vermindert ist oder augenscheinlich völlig fehlt. Die . weiterführenden Untersuchungen zur Natur dieses Bestandteils, die nachfolgend genauer beschrieben werden, ergaben, dass es sich bei dem signifikant verminderten Bestandteil um Apolipoprotein C-I handelt. Anschließende weitere Untersuchungen, die ebenfalls nachfolgend noch genauer beschrieben werden, führten dann zu dem weiteren überraschenden Ergebnis, dass Befunde, die bei einer ersten vorläufigen Auswertung für eine Parallelität der Veränderungen bei Sepsis- und Krebspatienten sprachen, in Wirklichkeit klar gegeneinander abzugrenzen sind und es ermöglichen, durch Messung von Apolipoprotein C-I in Serum oder Plasma von humanen Patienten Sepsiserkrankungen deutlich von Krebserkrankungen zu unterscheiden, was als solches eine Erkenntnis darstellt, die für diagnostische Zwecke nutzbar gemacht werden kann.

Es kann als Aufgabe der vorliegenden Erfindung bezeichnet werden, einen neuen, für diagnostische Bestimmungen zur Erkennung von Krebserkrankungen geeigneten proteinischen Biomarker aufzufinden und für diagnostische Zwecke nutzbar zu machen, dessen mengenmäßiges Auftreten in humanem Serum oder Plasma sich folgendermaßen von den bei augenscheinlich gesunden Normalpersonen gefundenen Werten unterscheidet: die Apolipoprotein C-I-Immunreaktivität ist in der Probe gegenüber gesunden Kontrollpersonen erhöht und der Anteil an Apolipoprotein C-I, das an hydrophobe Molekülstrukturen bindet, ist gegenüber gesunden Kontrollpersonen signifikant vermindert, sodass, die mit einem Immunoassay in der Probe direkt bestimmte Apolipoprotein C-I Konzentration und die Apolipoprotein C-I Konzentration in derjenigen Probe, die mit einem Adsporptionsmittel mit hydrophoben Oberflächen behandelt wurde, vergleichbare Werte aufweisen und dessen Bestimmung daher ein wertvolles diagnostisches Hilfsmittel darstellt.

Diese Aufgabe wird durch ein Verfahren gemäß Anspruch 1 gelöst. Bevorzugte Ausgestaltungen, sind in den Unteransprüchen angegeben.

Wenn in der vorliegenden Anmeldung bzw. den Ansprüchen als zu bestimmende Spezies neben Apolipoprotein C-I auch "dessen Abkömmlinge" erwähnt werden, so sind darunter insbesondere Fragmente und Aggregate zu verstehen, insbesondere solche, die sich bei dem jeweiligen gewählten Bestimungsverfahren wie das freie Protein Apolipoprotein C-I verhalten. Die "Abkömmlinge" können z.B. um einzelne Aminosäuren oder Aminosäuresequenzen verkürzte Apolipoprotein C-I-Moleküle sein, oder auch - z.B. durch Aggregation - sterisch oder konformationell veränderte vollständige Apolipoprotein C-I-Moleküle.

Die neuen Erkenntnisse, die die Verwendung von Apolipoprotein C-I als Biomarker ermöglichen, besagen, daß bei Patienten, die an Krebserkrankungen leiden, die erfaßbare Menge an Apolipoprotein C-I, das an hydrophobe Molekülstrukturenbindet, gegenüber augenscheinlich gesunden Normalpersonen stark erniedrigt ist. Daraus läßt sich der Schluß ziehen, daß bei den genannten Erkrankungen besagtes Apolipoprotein C-I in unzureichenden Mengen gebildet, verbraucht und/oder anormal gebunden wird, was sich im Vergleich mit Gesunden als Mangel an Apolipoprotein C-I interpretieren läßt.

Alle eingangs und in den Ansprüchen, auf die Bezug genommen wird, wiedergegebenen Sachverhalte und zu schützenden Lehren werden nachfolgend unter Bezugnahme auf experimentelle Daten und zugehörige Figuren noch näher erläutert, wobei gleichzeitig die Bedeutung einzelner, in der vorliegenden Beschreibung verwendeter Begriffe noch ergänzend erläutert wird. Auf die nachfolgenden Ausführungen wird daher zur Auslegung der Patentansprüche und zur Präzisierung der bisherigen allgemeinen Ausführungen ausdrücklich Bezug genommen.

In den Figuren zeigen:

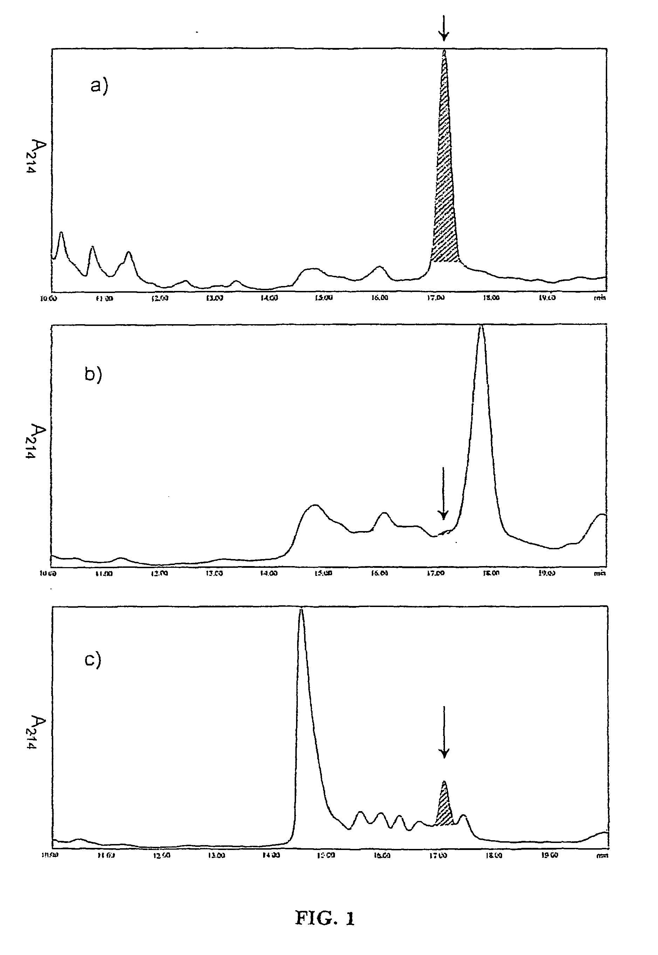

- Fig. 1: die Elutionsprofile der Reversphasen C18 HPLC von Serum- bzw. Plasma-Fraktionen, die mit Essigsäure (50 mM) aus Octylsepharose-Chromatographiesäulen eluierbar waren, die mit Seren bzw. Plasmen von (a) augenscheinlich gesunden Normalpersonen, (b) Pa- tienten mit verschiedenen malignen Tumor-Erkran- kungen und (c) von Sepsis-Patienten beschickt worden waren, nach einem zwischengeschalteten Waschen der beladenen Säulen mit phosphatgepufferter Salzlösung (PBS).

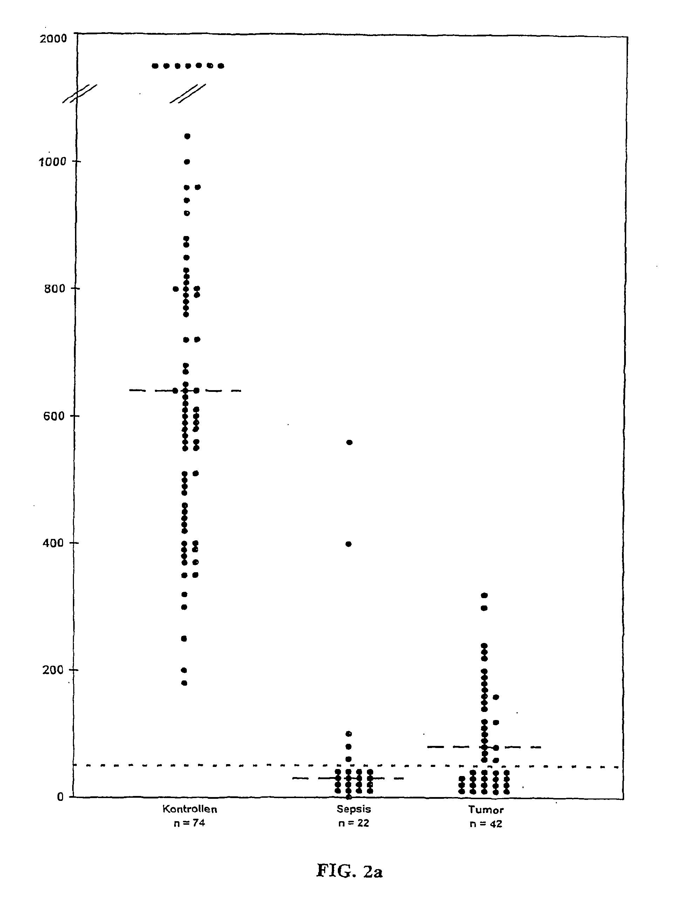

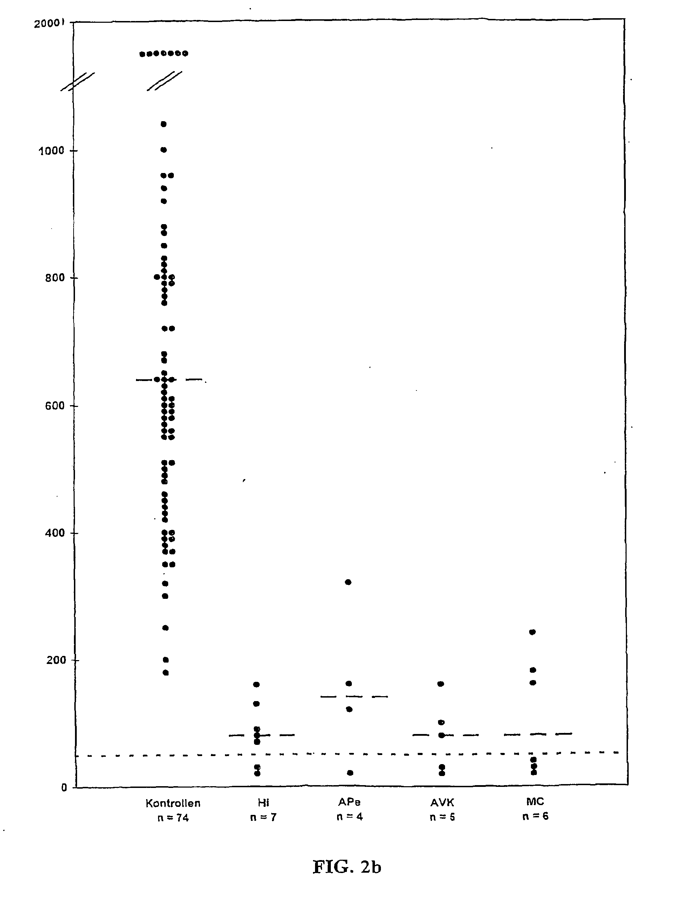

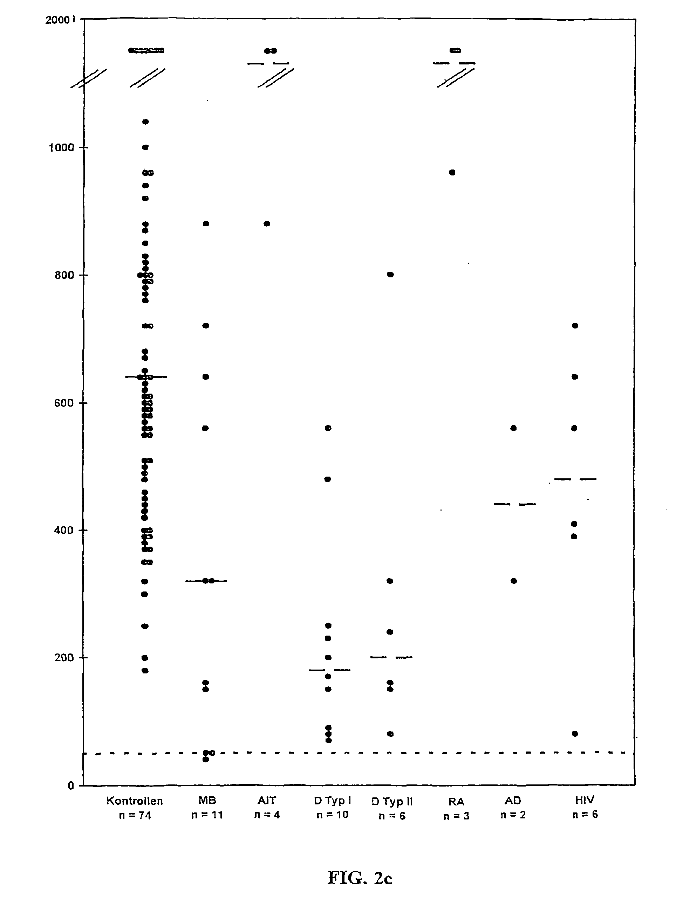

- Fig. 2: durch Peakintegration von HPLC-Elutionsprofilen des in Fig. 1 gezeigten Typs erhaltene Relativmengen an eluierbarem Apolipoprotein C-I in Proben von augen- scheinlich gesunden Normalpersonen und Patienten, die an Erkrankungen unterschiedlichen Typs litten (Sepsis, Tumor, Herzinfakt, Angina Pektoris, arte- rielle Verschlußerkrankungen, Morbus Crohn, Morbus Basedow, Autoimmunthyreoiditis, Diabetes Typ I, Diabetes Typ II, rheumatoide Arthritis, Alzheimer Krankheit, HIV positive Seren; jeweils unabhängig klinisch abgesicherte Diagnosen).

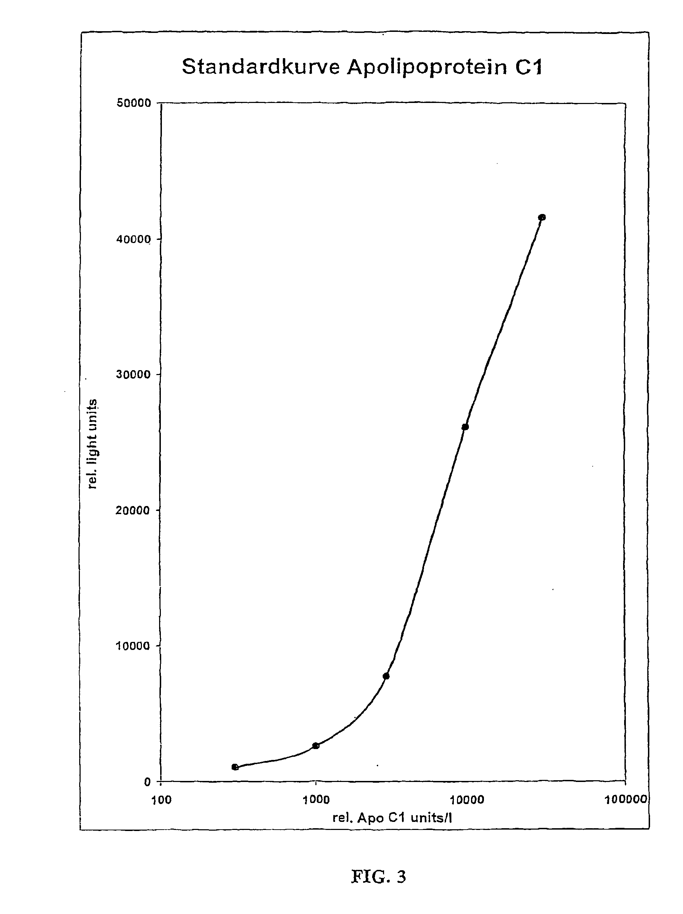

- Fig. 3: die Standardkurve eines mit zwei affinitäts- gereinigten polyklonalen Apolipoprotein C-I-Anti- körpern arbeitenden Sandwich-Immunoassays, wie sie unter Verwendung von synthetischem Apolipoprotein C- I als Standard erhalten wird.

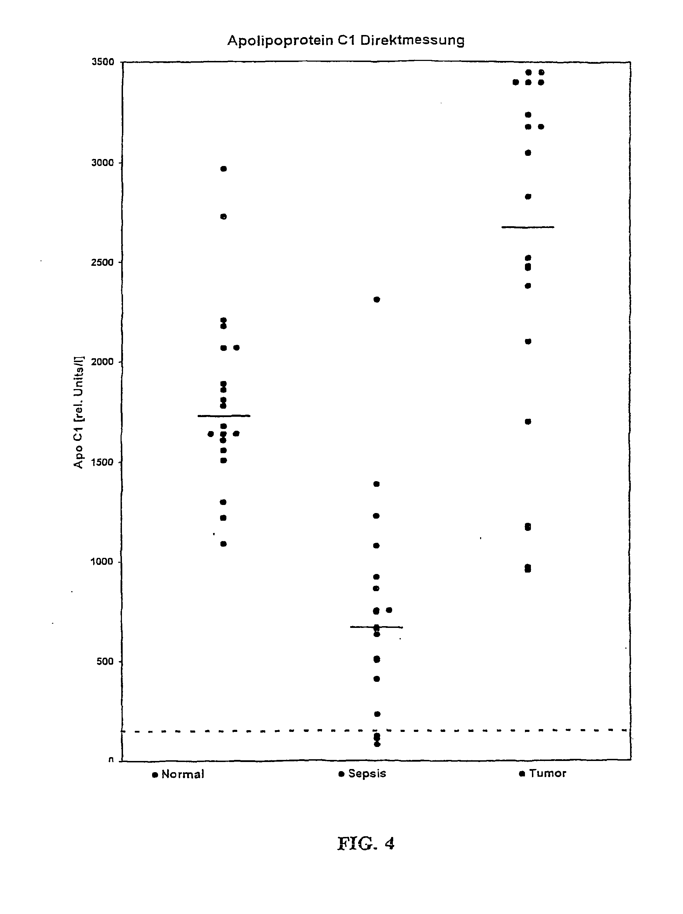

- Fig. 4: die Ergebnisse der direkten Bestimmung von Apolipo- protein C-I in Seren und Plasmen von augenscheinlich gesunden Normalpersonen, Sepsis-Patienten und Tumor- Patienten unter Verwendung eines Apolipoprotein C-I- Sandwich-Immunoassays, von dem eine typische Eich- kurve in Fig. 3 gezeigt ist; und

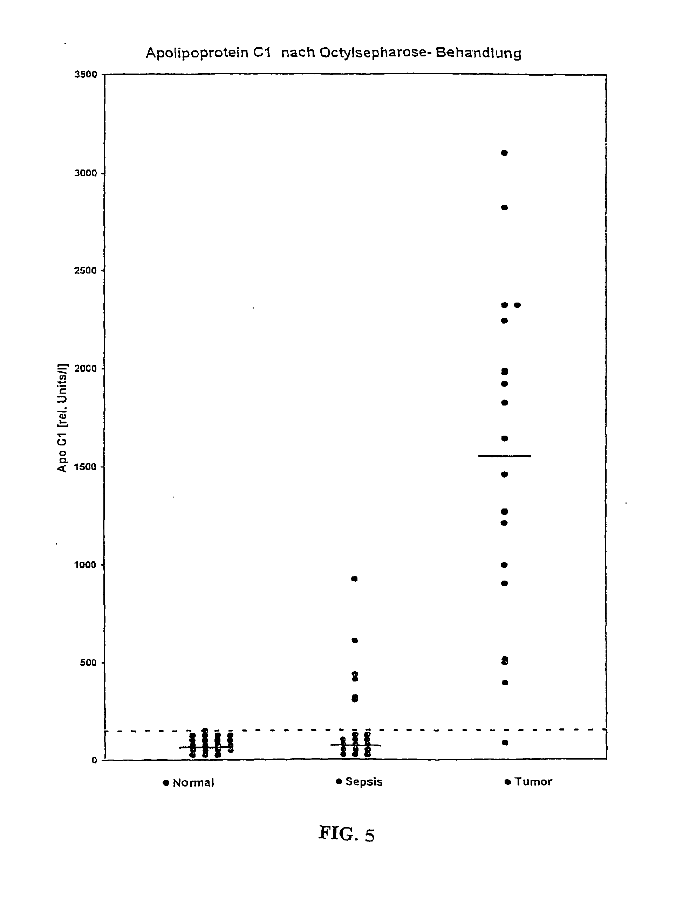

- Fig. 5: die Ergebnisse einer Wiederholung der Messungen, deren Ergebnisse in Fig. 4 gezeigt sind, nachdem die jeweiligen Proben mit Octylsepharose behandelt worden waren.

### Beschreibung der durchgeführten Versuche und der erhaltenen Ergebnisse

### A. CHROMATOGRAPHISCHE UNTERSUCHUNGEN

### 1. Gewinnung von Serum- oder Plasma-Fraktionen, die die an hydrophobe Oberflächen bindenden und von diesen eluierbaren Bestandteile von humanem Serum/Plasma enthalten

Proben von jeweils 0, 5 ml von Seren/Plasmen von augenscheinlich gesunden Normalpersonen und von Patienten, die an einer Reihe von unabhängig klinisch diagnostizierten Erkrankungen litten, wurden mit 0,5 ml Octylsepharose-Chromatographiematerial (Bezugsquelle: Pharmacia; 0,25 ml gepacktes Material, gewaschen in PBS) in PBS vermischt und 10 min bei Raumtemperatur bei leichtem Schütteln inkubiert. Anschließend wurde die Mischung in eine Polypropylensäule (Durchmesser 5 mm) gefüllt, und durch Waschen mit 20 ml PBS wurden die ungebundenen Substanzen von den an das Octylsepharosematerial gebundenen Substanzen getrennt.

Die Desorption der an das Octylsepharosematerial gebundenen Substanzen (Proteine) erfolgte durch Elution mit 50 mM Essigsäure (pH 2,5).

### 2. Reversphasen C18 HPLC

Das aus den Octylsepharosesäulen gewonnene Essigsäureeluat wurde jeweils direkt mittels Reversphasen C18 HPLC über eine µ Bondapak C18-Säule (3,9 x 300 mm, Millipore, Waters) analysiert.

Für die HPLC-Chromatographie wurden Gradienten aus einem Laufmittel A (Mischung aus 95 Vol.-Teilen Wasser, 5 Vol.-Teilen Acetonitril, 0,1 Vol.-Teil Trifluoressigsäure) und einem Laufmittel B (10 Vol.-Teile Wasser, 90 Vol.-Teile Acetonitril, 0,1 Vol.-Teile Trifluoressigsäure) verwendet.

Nach dem Auftragen des Essigsäureeluats auf die HPLC-Säule erfolgte die Analyse in folgendem Gradienten aus Laufmittel A/Laufmittel B:
t = 0-5 min von 100/0 (A/B) nach 65/35 (A/B)
t = 5-20 min von 65/35 (A/B) nach 40/60 (A/B)
t= 20-22 min von 40/60 (A/B) nach 0/100 (A/B).

Die Durchflußgeschwindigkeit betrug 1 ml/min. Der Säulenausfluß wurde kontinuierlich auf Absorption bei 214 nm vermessen.

Fig. 1 zeigt drei typische Elutionsprofile für Fraktionen aus Proben von augenscheinlich gesunden Normalpatienten (a), von Patienten mit unterschiedlichen Tumorerkrankungen (b), und von Patienten mit Sepsis (c).

Überraschend ,zeigte sich, daß die HPLC-Elutionsprofile der genannten Personengruppen sich deutlich und systematisch von einander unterschieden. Die für die vorliegende Anmeldung entscheidende Bande ist dabei diejenige, die bei 17,05 min eluiert wird.

Diese Bande wurde mittels Peakintegration für alle untersuchten Serum- und Plasmaproben relativ quantifiziert, und die auf diese Weise ermittelten relativen Substanzmengen für eine Vielzahl von Kontroll- und Patientenproben sind graphisch in Abbildung 2 dargestellt. Im einzelnen wurden folgende Proben untersucht:

42 Proben von Tumor-Patienten (9 x Darmkrebs, 7 x Bronchialkarzinom, 13 x Mammakarzinom, 8 x Ovarialkarzinom, 5 x Pankreaskarzinom), 22 Proben von Sepsis-Patienten, 7 Proben von Herzinfakt-Patienten (Hi), 4 Proben von Patienten mit Angina Pektoris (APe), 5 Proben von Patienten mit arteriellen Verschlußkrankheiten (AVK), 6 Proben von Patienten mit Morbus Crohn (MC), 11 Proben von Patienten mit Morbus Basedow, 4 Proben mit Patienten mit Autoimmunthyreoiditis (AIT), 10 Proben von Patienten mit Diabetes Typ I (D-Typ 1), 6 Proben mit Patienten mit Diabetes Typ II (D-Typ II), 3 Proben von Patienten mit rheumatoider Arthritis (RA), 2 Proben von Patienten mit Alzheimer (AD) und 6 Proben von HIV-positiven Patienten).

Wie Fig. 2 sofort entnehmbar ist, werden für Proben von gesunden Normalpersonen deutlich positive Werte für die Substanz gefunden, die dem vermessenen Peak bei 17,05 min entspricht. In den Proben Erkrankter, und zwar ganz besonders deutlich in den Proben von Sepsis-Patienten, Tumor-Patienten, Patienten mit den manifesten/akuten Herzerkrankungen (Hi, APe, AVK) sowie Patienten mit Morbus Crohn werden hoch signifikant erniedrigte Anteile der gleichen Substanz gemessen. Auch für Patienten mit Diabetes Typ I und Diabetes Typ II werden gegenüber Gesunden signifikant, wenn auch nicht im gleichen Maße erniedrigte Werte gefunden. Für Patienten mit Morbus Basedow, Autoimmunthyreoidis, rheumatoider Arthritis, Alzheimer-Krankheit sowie für HIV-positive Patienten sind die entsprechenden Konzentrationsabweichungen geringer bzw. statistisch nicht signifikant.

Die für die einzelnen Proben-Gruppen erhaltenen Mittelwerte der gefundenen Relativmengen sind in der nachfolgenden Tabelle zusammengefaßt:

| Probenart | Mittelwert (relative Absorptionseinheiten) |
|---|---|
| Kontrollen | 640 |
| Sepsis | 30 |
| Tumor | 80 |
| Herzinfakt | 80 |
| Angina Pektoris | 140 |
| Arterielle Verschlußerkankungen | 80 |
| Morbus Crohn | 80 |
| Morbus Basedow | 320 |
| Autoimmunthyreoiditis | 1130 |
| Typ I Diabetes | 180 |
| Typ II Diabetes | 200 |
| Rheumatoide Arthritis | 1130 |
| Alzheimer | 440 |
| HIV positiv | 480 |

### 3. Identifizierung der zur Proben-Charakterisierung verwendeten Bande (bei 17,5 min eluierende Bande)

Zur Identifizierung wurde die dieser Bande entsprechende eluierte Substanz aufgefangen, getrocknet (speed vac) und einer Peptid-Analyse unterzogen. Bei einer Sequenz-Analyse des N-Terminus wurde die Sequenz TPDVS ermittelt. Das mittels Massenspektrum (ESI MS) ermittelte Molekulargewicht beträgt 6630 ± 15 D. Diese Ergebnisse korrelierten eindeutig mit den bekannten Daten für das humane Apolipoprotein C-I (vgl. z.B. (3)), das die Peptid-Sequenz gemäß SEQ ID No.1 aufweist (vgl. auch SWISS PROT Accession No. 3660227) und ein theoretisches Molekulargewicht von 6627 D aufweist.

Nach der Trypsin-Verdauung der Substanz und anschließender Massenspektroskopie (ESI MS; "Trypsin-Fingerprinting") bestätigte sich die getroffene Zuordnung vollständig.

Das auf die beschriebene chromatographische Arbeitsweise bestimmbare Apolipoprotein C-I wird im Rahmen der vorliegenden Anmeldung auch als "freies Apolipoprotein C-I" bezeichnet. Es weist die Eigenschaften auf, aus einer Serum- oder Plasma-Probe (in PBS Verdünnung) heraus an hydrophobe Molekülstrukturen, z.B. die Octylreste eines hydrophoben Octylsepharose-Chromatographier-Materials, gebunden zu werden, von dem es unter typischen Bedingungen für die Elution von Proteinen, insbesondere mit verdünnter Essigsäure, eluiert werden kann. Die Verwendung des Begriffs "freies Apolipoprotein C-I" bedeutet jedoch nicht notwendigerweise, daß das durch hydrophobe Interaktionschromatographie abtrennbare material in den Ursprungsproben völlig frei vorliegen muss. Assoziate bzw. Aggregate, auch mit Lipiden, die unter den Versuchsbedingungen beim Kontakt mit Octylsepharose unter Bindung des Apolipoproteins C-I an das Chromatographiermaterial aufgebrochen werden, sind ebenfalls als "freies Apolipoprotein C-I" im Sinne der Verwendung dieses Begriffs in der vorliegenden Anmeldung anzusehen.

Die "hydrophoben Molekülstrukturen" können somit auch Teile der Oberfläche eines hydrophoben Chromatographiematerials sein. Sie können aber auch einzelne Moleküle mit hydrophoben Strukturbereichen sein, über die eine Bindung eines solchen Moleküls an Apolipoprotein C-I,erfolgen kann, z.B. Moleküle mit hydrophoben Strukturen, die markiert werden können und z.B. in homogenen Assays zur Markierung von Apolipropotein C-I in einer Probenflüssigkeit dienen können. Die Fähigkeit zur Bindung an "hydrophobe Molekülstrukturen" ist dann vorhanden, wenn die für eine solche Bindung zur Verfügung stehenden Bereiche des Apolipoproteins C-I in der Probe nicht durch andere Substanzen blockiert sind, z.B. die Lipide in Lipoproteinen. Selbst wenn z.B. davon gesprochen wird, dass man solche Anteile an Apolipoprotein C-I bestimmt, die an Octylsepharose binden, bedeutet das nicht, dass man bei der Bestimmung tatsächlich eine Bindung an Octylsepharose ausnutzen muss. Eine solche Angabe ist vielmehr als Charakterisierung der zu bestimmenden Substanz zu verstehen, auch wenn diese nach ganz anderen Verfahren bestimmt wird. Z.B. kann das entsprechende Apolipoprotein C-I das gleiche sein, das auch, wie nachfolgend beschrieben, mittels eines Immunoassays bestimmt wird.

### B. IMMUNDIAGNOSTISCHE DIREKTBESTIMMUNGEN VON APOLIPOPROTEIN C-I IN SEREN/PLASMEN

### 1. Apolipoprotein C-I-Immunoassay

Für die direkte immundiagnostische Bestimmung von Apolipoprotein C-I im Serum wurde aus den nachfolgend beschriebenen Bestandteilen ein Immunoassay vom Sandwich-Typ aufgebaut:
a) Coated Tubes: Polystyrolröhrchen (Greiner) wurden mit einem kommerziell erhältlichen polyklonalen affinitätsgereinigten Antikörper gegen Apolipoprotein C-I (Bezugsquelle: Acris Antibody, Bad Neuheim, Deutschland) beschichtet. Der Antikörper war nach Herstellerangaben durch Immunisierung von Kaninchen mit humanen Apo C-I erhalten und über eine Sepharose-Affinitätssäule mit humanem Apolipoprotein C-I aufgereinigt worden. Zur Beschichtung wurden 0,2 µg Antikörper in 300 µl PBS an Polystyrolröhrchen (Greiner, Deutschland) gebunden, die mit Schaf-Anti-Kaninchen IgG-Antikörper (Sigma) beschichtet waren. Die Bindung wurde nach 18 Stunden bei Raumtemperatur beendet. Anschließend wurden die Röhrchen 2 mal mit je 3 ml 0,5% Rinderserumalbumin (BSA) in PBS gewaschen. Nach ihrer Trocknung in Vakuum wurden die Röhrchen als Festphase für den Apolipoprotein C-I-Immunoassay verwendet.
b) Akridiniumester-markierter Antikörper: 100 µg eines anderen Antikörpers gegen humanes Apolipoprotein C-I (aus Kaninchen; Bezugsquelle: Academie Bio-Medical Company, Texas, USA) in 100 µl PBS wurden mit 10 µg Akridinium-NHS-Ester (in 10 µl Acetonitril) umgesetzt. Nach einer 10-minütigen Inkubation bei Raumtemperatur erfolgte die Reinigung des markierten Antikörpers unter Abtrennung unumgesetzter Bestandteile der Reaktionsmischung durch HPLC an SW 300 (Waters). Für seine Verwendung im Immunoassey wurde der markierte Antikörper in PBS mit 0,5% BSA und 1 mg/ml Kaninchen IgG zur Absättigung der Röhrchenwände auf ca. 1 Mio. RLU/300 µl (RLU = relative Lichteinheiten) eingestellt.

### 2. Durchführung der immundiagnostischen Bestimmung von Apolipoprotein C-I in Serum- bzw. Plasma-Proben

Serum- bzw. Plasmaproben wurden 1:10 000 mit PBS, 0,5% BSA verdünnt. Davon wurden jeweils 300 µl in die mit dem immobilisierten Antikörper beschichteten o.g. Röhrchen pipettiert und anschließend für 4 Stunden bei Raumtemperatur unter Schütteln (300 U/min auf einem Heidolph-Kreisschüttler) inkubiert. Der Röhrcheninhalt wurde anschließend mit PBS ausgewaschen (4-maliges Befüllen und Dekantieren mit jeweils 1 ml PBS), und an die Röhrchenwand gebundenes Apolipoprotein C-I wurde innerhalb von 20 Stunden bei Raumtemperatur und 300 U/min mit 300 µl je Röhrchen des akridiniumestermarkiertenAnti-Apolipoprotein C-I-Antikörpers umgesetzt. Danach wurde ungebundener markierter Antikörper durch 5-maliges Waschen mit je 1 ml PBS entfernt, und die verbleibende Chemilumineszenz wurde auf bekannte Weise in einem Berthold Luminometer 952 T gemessen.

Zur Kalibrierung des Assays wurde ein synthetisches Apolipoprotein C-I verwendet. Eine typische Standardkurve, wie sie für den obigen Assay erhalten wird, ist in Fig. 3 gezeigt.

### 3. Ergebnisse.

Mit Hilfe des beschriebenen Immunoassays vom Sandwich-Typ wurden Kontrollseren von augenscheinlich gesunden Normalpersonen, wie sie auch bei den chromatographischen Untersuchungen verwendet wurden, sowie Sepsis und Tumorproben vermessen. Die Messungen der Proben von Sepsis-Patienten bestätigen im wesentlichen die Ergebnisse der HPLC-Untersuchungen, indem für Apolipoprotein C-I gegenüber Normalpersonen signifikant verminderte Meßwerte erhalten wurden. Bei der Messung von Proben von Tumor-Patienten, für die bei den chromatographischen Untersuchungen ebenfalls verminderte Konzentrationen ermittelt wurden, wurden jedoch tendenziell erhöhte Meßwerte erhalten. Die Ergebnisse sind in Fig. 4 graphisch dargestellt.

Zur Überprüfung der Frage, ob die unterschiedlichen Ergebnisse für Tumorpatienten bei den chromatographischen Bestimmungen einerseits und den immundiagnostischen Bestimmungen andererseits auf den vorausgegangenen Selektionsschritt mittels hydrophober Interaktionschromatographie zurückzuführen war, wurden die im Immunoassay gemessenen Proben vor einer weiteren Bestimmung zur Bindung "freier" Anteile an Apolipoprotein C-I mit Octylsepharose behandelt.

### 4. Behandlung der Proben mit Octylsepharose

150 µl von den gleichen Serum- bzw. Plasmaproben, die wie beschrieben im Immunoassay vermessen worden waren, wurden mit 200 µl Octylsepharose (Pharmacia, 50 µl Gel in PBS) vermischt, für eine Stunde bei Raumtemperatur unter leichtem Schütteln inkubiert, wonach die Octylsepharose mit daran gebundenen Apolipoprotein C-I-Anteilen durch Zentrifugieren (15 min bei 2.000 G) abgetrennt wurde. Der erhaltene Überstand wurde 1:5000 in PBS/0,5% BSA verdünnt und anschließend, wie oben beschrieben, in dem Immunoassay vermessen.

Die erhaltenen Ergebnisse sind in Fig. 5 gezeigt. Es ist zu erkennen, daß durch die Behandlung mit Octylsepharose das gesamte "freie" Apolipoprotein C-I, das bei der Messung mit dem beschriebenen Immunoassay vom Sandwich-Typ erfaßbar ist, an die Octylsepharose gebunden worden war, so dass im Überstand kein Apolipoprotein C-I mehr nachweisbar war. Ähnliche Beobachtungen wurden für die behandelten Sepsis-Proben gemacht, indem der größte Anteil der im Vergleich zu den Normalproben bereits stark verminderten Mengen an bestimmbarem Apolipoprotein C-I durch die Behandlung mit der Octylsepharose aus der Probe entfernt wurde.

Überraschenderweise wurde das in einem Immunoassay der beschriebenen Art bestimmbare Apolipoprotein C-I durch die Behandlung mit Octylsepharose jedoch nicht aus den Proben von Tumorkranken entfernt.

### C. DISKUSSION

Die geschilderten Befunde zeigen, daß wenigstens ein großer Anteil des Apolipoproteins C-I in Seren bzw. Plasmen von Tumor-Patienten in einer Form vorliegt, in der es seine Bindefähigkeit an hydrophobe Oberflächen verloren hat, in einem Immunoassay als Immunreaktivität jedoch noch bestimmbar ist. Eine mögliche Erklärung für diese Beobachtungen wäre, daß bei dem in Seren von Tumorpatienten vorkommenden Apolipoprotein C-I die für hydrophobe Wechselwirkungen zur Verfügung stehenden Molekülbereiche abgesättigt bzw. blockiert sind, und zwar durch Bindungspartner, die von der Octylsepharose nicht verdrängt werden können. Die Natur dieser Bindungspartner ist derzeit noch unbekannt. Es ist jedoch nicht auszuschließen, daß es sich um tumorspezifische Substanzen handelt, die als solche oder aufgrund der Inaktivierung hydrophober Bindungspartner, wie z.B. von Apolipoprotein C-I, für die Tumorentwicklung eine wichtige Rolle spielen, z.B. durch eine negative Beeinflussung der natürlichen Immunantwort oder durch aktive Förderung des Tumorwachstums. Obwohl in den auf immundiagnostischem Wege untersuchten Proben keine signifikante Verminderung des Apolipoprotein C-I-Anteils feststellbar ist, zeigen die parallelen chromatographischen Untersuchungen, daß dieses nicht im o.g. Sinne "frei", sondern in einer anderen Form mit verminderter Bindefähigkeit an hydrophobe Bindungspartner vorliegt. "freies", d.h. an hydrophobe Bindungspartner bindendes Apolipoprotein C-I ist dagegen, wie bei den anderen Erkrankungen wie z.B. Sepsis, in Seren von Tumorpatienten vermindert.

Es kann aufgrund der geschilderten Befunde vermutet werden, daß es für Tumorpatienten vorteilhaft ist, die bei ihnen gestörte Balance im Sinne eines verminderten Anteils an "freiem" Lipoprotein C-I durch externe Zufuhr von Apolipoprotein C-I auszugleichen, und damit das Kranheitsgeschehen positiv zu beeinflussen, z.B. indem die tumortypischen Bindungspartner durch externes Apolipoprotein C-I abgesättigt werden und zusätzliches freies Apolipoprotein C-I bereitgestellt wird.

Eine ähnliche Annahme, nämlich daß eine externe Zufuhr von Apolipoprotein C-I zum Ausgleich eines gestörtes Spiegels an "freiem" Apolipoprotein C-I vorteilhaft ist, gilt auch für die anderen Erkrankungen, z.B. den Fall einer Sepsis. Ist bei Sepsis die Produktion von Apolipoprotein C-I gestört, kann eine externe Zufuhr einen dadurch bedingten Mangel ausgleichen. Ist die Verminderung an "freiem" Apolipoprotein C-I im Serum/Plasma eines Patienten bei Sepsis darauf zurückzuführen, daß das Apolipoprotein C-I durch Bindung an pathologische Gewebsstrukturen oder ggf. unlösliche bakterielle Strukturen aus dem Kreislauf entfernt wird, kann eine externe Zufuhr von Apolipoprotein C-I den entstandenen Mangel ausgleichen bzw. eine Absättigung der Bindungsstellen im pathologischen Gewebe oder an den Bakterienstrukturen bewirken. Durch Überwachung der durch externe Zugabe erhöhten Konzentration an freiem Apolipoprotein bzw. als Immunreaktivität bestimmbarem Apolipoprotein in Blutproben des behandelten Patienten kann die jeweils erforderliche Menge auf einfache Weise überwacht werden.

Damit macht die vorliegende Erfindung auch eine neuartige therapeutische Nutzung von Apolipoprotein C-I, oder von geeigneten Derivaten oder sein physiologisches Verhalten simulierenden Verbindungen zur Behandlung von Krebs, Sepsis und anderen Erkrankungen, die mit einer Verminderung des nachweisbaren freien Apolipoproteins C-I gegenüber Gesunden verbunden sind, möglich.

Literaturliste:
1. Erika Polz et al., Human Apolipoprotein C-I: Concentration in Blood Serum and Lipoproteins, Biochemical Medicine 24, 229-237 (1980)
2. Kane, J.P., in "Lipid Metabolism in Mammals" (F.Snyder, Ed.), Vol.1, S. 209, Plenum, New York, 1.977
3. Shulman, RS et al., The complete amino acid sequence of C-I (apoLP-Ser), an apolipoprotein from human very low density lipoproteins, J Biol Chem. 250, 182-190 (1975)
4. Xiao-Ren Pan et al., Abnormal Composition,of Apolipoproteins C-I, C-II, and C-II in Plasma and Very-Low-Density Lipoproteins of Non-Insulin-Dependent Diabetic Chinese, Clin. Chem. 32, No.10, 1914-1920 (1986)
5. Jutta Poensgen, Apolipoprotein C-I inhibits the hydrolysis by phospholipase A2 of phospholipids in liposomes and cell membranes, Biochim.Biophys.Acta, 1042 (1990), 188-192
6. Ming Liu et al., Activation of plasma lysolecithin acyltransferase reaction by apolipoproteins A-I, C-I and E, Biochim.Biophys.Acta, 1168 (1993), 144-152
7. Nina D. Bren et al., Quantification of Human Plasma Apolipoproteins C-I, C-II, and C-III by Radioimmunoassays, Mayo Clin Proc, July 1993, Vol.68, 657-664
8. Rampratap S. Kushwaha et al., Characterization'of cholesteryl ester transfer protein inhibitor from plasma of baboons (Papio sp.), J.Lipid Res. 1993, 34:1285-1297
9. N. Savion et al., Role of apolipoproteins A-I, A-II and C-I in cholesterol efflux from endothelial and smooth muscle cells, Eur Heart J (1993) 14, 930-935
10. Janine H. van Ree et al., Inactivation of Apoe and Apoc1 by two consecutive rounds of gene targeting: effects on mRNA expression levels of gene cluster members, Human Molecular Genetics, 1995, Vol.4, No.8, 1403-1409
11. Miek C. Jong et al., Role of ApoCs in Lipoprotein Metabolism - Functional differences Between ApoC1, ApoC2, and ApoC3, Arterioscler Thromb Vasc Biol.1999; 19:472-484
12. Thomas Gautier et al., Human Apolipoprotein C-I Accounts for the Ability of Plasma High Density Liproproteins to Inhibit the Cholesteryl Ester Transfer Protein Activity, J. Biol. Chem. 275, No.48, 37504-37509, (2000)
13. Jong M.C. et al., Insights into Apolipoprotein C Metabolism from Transgenic and Gene-Targeted Mice, Int.J.Tissue React. XXII (2/3) 59-66 (2000)
14. Neil S. Shachter, Apolipoproteins C-I and C-III as important modulators of lipoprotein metabolism, Current Opinion in Lipidology 2001, 12:297-304
15. Benjamin W. Atcliffe et al., The interaction of human apolipoprotein C-I with sub-micellar phospholipid, Eur. J. Biochem. 268, 2838-2846 (2001)
16. Thomas Gautier et al., Apolipoprotein CI Deficiency Markedly Augments Plasma Lipoprotein Changes Mediated by Human Cholesteryl Ester Transfer Protein (CETP) in CETP Transgenic/ApoCI-knocked Out Mice, J. Biol. Chem. 277, No.35, 31354-31363 (2002)
17. Puiying A. Mak et al., Regulated Expression of the Apolipoprotein E/C-I/C-IV/C-II Gene Cluster in Murine and Human Macrophages, J. Biol. Chem. 277, No.35, 31900-31908 (2002)
18. Johan Björkegren et al., Postprandial Enrichment of Remnant Lipoproteins With ApoC-I in Healthy Normolipidemic Men With Early Asymptomatic Atherosclerosis, Arterioscler Thromb Vasc Biol. 2002; 22:1470-1474
19. John G. Raynes et al., Purification of Serum Amyloid A and Other High Density Apolipoproteins by Hydrophobic Interaction Chromatography, Analytical Biochemistry 173, 116-124 (1988)
20. Lothar Thomas (Herausgeber): Labor und Diagnose, 5. erweiterte Auflage, Frankfurt 200, Kapitel 4 Fettstoffwechsel, S.177-190
21. Cesare R. Sitori, Evaluation of Lipoproteins/Apolipoproteins as Therapeutic Agents for the Treatment of Vascular and Nonvascular Disease, Am J Cardiol., Vol 81, 36F-39F (1998)

## Patentansprüche

1. Verfahren zur Diagnose, Früherkennung" Risikoabschätzung sowie zur Verlaufskontrolle yon Krebserkrankungen, **dadurch gekennzeichnet, dass** man
- in einer Serum- oder Plasmaproben eines humanen Patienten denjenigen Anteil des gesamten in einer Probe vorhandenen Apolipoproteins C-I bestimmt, der (i) durch direkte Bestimmung mit einem Immunoassay von Sandwichtyp erfaßbar ist ("Apolipoprotein C-I-Immunreaktivität") und (ii) dessen Messwert durch Behandlung der Probe mit einem Adsorptionsmittel mit hydrophoben Oberflächen nicht oder nicht signifikant vermindert wird, und
- dass man auf das Vorliegen einer Krebserkankung schließt, wenn
- die Apolipoprotein C-I Immunreaktivität in der Probe gegenüber gesunden Kontrollpersonen erhöht ist und der Anteil an Apolipoprotein C-I, das an hydrophobe Molekül-. Strukturen bindet, gegenüber gesunden Kontrollpersonen signifikant vermindert ist, so dass
- die mit dem Immunoassay in der Probe direkt bestimmte Apolipoprotein C-I Konzentration und die Apolipoprotein C-I Konzentration in derjenigen Probe, die mit einem Adsorptionsmittel mit hydrophoben Oberflächen behandelt wurde, vergleichbare Werte aufweisen.

2. Verfahren nach Anspruch 1, **dadurch gekennzeichnet, dass** man zur Behandlung der Probe mit einem Adsorptionsmittel mit hydrophoben Oberflächen eine Serum- oder Plasmaprobe einer hydrophoben Interaictions-Chromatographie unterwirft, bei der freies Apolipoprotein C-I an das Chromatographiematerial gebunden wird.

3. Verfahren nach Anspruch 2, **dadurch gekennzeichnet, dass** man als Chromatographiematerial Octylsepharose verwendet.

## Claims

1. A method for the diagnosis, early detection, risk estimation and monitoring of the course of cancer diseases, **characterized in that**
- in a serum or plasma sample from a human patient that fraction of the total apolipoprotein C-I present in a sample is determined (i) which is detectable by direct determination using an immunoassay of the sandwich type ("apolipoprotein C-I immunoreactivity") and (ii) the measured value of which is not or not significantly reduced by a treatment of the sample with an adsorbent with hydrophobic surfaces, and
- that the presence of a cancer disease is concluded, when
- the apolipoprotein C-I immunoreactivity in the sample is elevated compared to healthy control individuals, and the proportion of apolipoprotein C-I, which binds to hydrophobic molecular structures, is significantly reduced compared to healthy control individuals such that
- the concentration of apolipoprotein C-I directly determined in the sample by the immunoassay, and the concentration of apolipoprotein C-I **in that** sample which has been treated with an adsorbent with hydrophobic surfaces have comparable values.

2. Method according to claim 1, **characterized in that** for the treatment of the sample with an adsorbent with hydrophobic surfaces a serum or plasma sample is subjected to a hydrophobic interaction chromatography in which free apolipoprotein C-I is bound to the chromatography material.

3. Method according to claim 2, **characterized in that** octylsepharose is used as chromatography material.

## Revendications

1. Procédé pour le diagnostic, la détection précoce, l'évaluation des risques, ainsi que pour le contrôle de l'évolution de maladies cancéreuses, **caractérisé en ce que**,
- dans un échantillon de sérum ou de plasma, issu d'un patient humain, on détermine la fraction de l'apolipoprotéine C-I, totale, présente dans un échantillon et (i) qui peut être détectée, par détermination directe, lors d'un essai immunologique du type sandwich (« Réactivité immunologique apolipoprotéine C-I »), et (ii) dont la valeur de mesure n'est pas réduite, ou ne l'est que dans une mesure non significative, par traitement de l'échantillon avec un moyen d'absorption, avec des surfaces hydrophobes, et
- que l'on conclue la présence d'une maladie cancéreuse quand
- la réactivité immunologique apolipoprotéine C-I est plus élevée dans l'échantillon que chez des sujets contrôles sains, et la quantité d'apolipoprotéine C-I, qui se lie à des structures moléculaires hydrophobes, est réduite de manière significative par rapport aux sujets contrôles sains, de sorte que
- la concentration d'apolipoprotéine C-I, déterminée directement dans l'échantillon au cours de l'essai immunologique, et la concentration d'apolipoprotéine C-I dans l'échantillon, qui a été traité avec un moyen d'absorption avec surfaces hydrophobes, présentent des valeurs comparables.

2. Procédé selon la revendication 1, **caractérisé en ce que**, pour le traitement avec un moyen d'absorption avec surfaces hydrophobes, on soumet un échantillon de
sérum ou de plasma à une chromatographie d'interaction hydrophobe, au cours de laquelle de l'apolipoprotéine C-I est liée à la matière chromatographique.

3. Procédé selon la revendication 2, **caractérisé en ce que** l'on utilise de l'octylsépharose en tant que matière chromatographique.


### SEQUENCE LISTING

<110> B.R.A.H.M.S Aktiengesellschaft
<120> Verfahren zur Diagnose von Erkrankungen unter Bestimmung von Apolipoprotein C-I, und dessen Verwendung in der Therapie von Erkrankungen
<130> 4118PDE
<140>
   <141>
<160> 1
<170> Patent In Ver. 2.1
<210> 1
   <211> 57
   <212> PRT
   <213> Homo sapiens
<400> 1
   Thr Pro Asp Val Ser Ser Ala Leu Asp Lys Leu Lys Glu Phe Gly Asn
    1               5                  10                  15
   Thr Leu Glu Asp Lys Ala Arg Glu Leu Ile Ser Arg Ile Lys Gln Ser
               20                  25                  30
   Glu Leu Ser Ala Lys Met Arg Glu Trp Phe Ser Glu Thr Phe Gln Lys
           35                  40                  45
   Val Lys Glu Lys Leu Lys Ile Asp Ser
       50                  55